# EUROPEAN PATENT APPLICATION

(11) **EP 4 442 293 A1**
(43) Date of publication of application: **09.10.2024**
(21) Application number: 24168607.0
(22) Date of filing: 05.04.2024
(51) Int. Cl.: A61M 1/36

(54) **PLATELET COLLECTION EMPLOYING MULTIPLE CENTRIFUGE ROTATION RATES**

(30) Priority: 07.04.2023 US 202363495078 P
(71) Applicant: Fenwal, Inc., Lake Zurich, IL 60047 (US)
(72) Inventor: KUSTERS, Benjamin E., Lake Zurich, 60047 (US); BROWN, Richard I., Lake Zurich, 60047 (US); MIN, Kyungyoon, Lake Zurich, 60047 (US)
(74) Representative: Maikowski & Ninnemann Patentanwälte Partnerschaft mbB

(57) **Abstract**

A biological fluid processing device includes pump and valve systems, a centrifuge and a controller. The controller is configured to operate the pump and valve systems to convey buffy coat into the centrifuge, operate the centrifuge at a first rotation rate to separate the buffy coat into platelets and red blood cells, operate the pump and valve systems to convey a first portion of the separated platelets from the centrifuge for collection, and operate the pump and valve systems to convey the separated red blood cells from the centrifuge for collection. The controller is further configured to operate the centrifuge at one or more second rotation rates lower than the first rotation rate, operate the pump and valve systems to convey the collected red blood cells into the centrifuge, and operate the pump and valve systems to convey a second portion of the platelets from the centrifuge for collection.

## Description

### Cross-Reference to Related Application(s)

This application claims the benefit of and priority to U.S. Provisional Patent Application Serial No. 63/495,078 filed on April 7, 2023, the contents of which are incorporated herein by reference.

### Field of the Disclosure

The present disclosure relates to centrifugal separation of a biological fluid and collection of separated fluid components. More particularly, the present disclosure relates to separating and collecting platelets from pooled buffy coats using multiple centrifuge rotation rates.

### Description of Related Art

Various blood processing systems now make it possible to collect particular blood constituents, instead of whole blood, from a blood source. Typically, in such systems, whole blood is drawn from a blood source, the particular blood component or constituent is separated, removed, and collected, and the remaining blood constituents are returned to the blood source. Removing only particular constituents is advantageous when the blood source is a human donor, because potentially less time is needed for the donor's body to return to pre-donation levels, and donations can be made at more frequent intervals than when whole blood is collected. This increases the overall supply of blood constituents, such as plasma and platelets, made available for transfer and/or therapeutic treatment.

According to one approach, whole blood may be separated into its constituents through centrifugation. This requires that the whole blood be passed through a centrifuge after it is withdrawn from, and before it is returned to, the blood source. To reduce contamination and possible infection (if the blood source is a human donor or patient), the blood is preferably processed within a sealed, sterile fluid flow circuit during the centrifugation process. The operator installs a fresh, sterile disposable flow circuit in the centrifuge before processing and removes and discards it afterwards. Typical disposable flow circuits are sealed and sterile, and include a separation chamber portion, which is mounted in cooperation on a durable, reusable assembly containing the hardware (centrifuge, drive system, pumps, valve actuators, programmable controller, and the like) that rotates the separation chamber and controls the flow through the fluid circuit. The separation chamber may be formed of a generally rigid material (e.g., molded plastic), in which case the chamber itself defines a flow path or channel in which blood is separated into two or more components, or a more flexible material (e.g., in the form of a belt or annulus), which relies upon the system hardware to support the chamber and define the shape of the chamber as blood flows through it.

With a disposable circuit loaded onto the centrifuge (or just prior to or during loading) the operator typically enters, for example, by means of a touch screen or other user interface system, a particular processing protocol to be executed by the system (e.g., a procedure wherein platelets are separated from whole blood and collected) and other parameters (e.g., the weight of the donor, the desired volume of separated blood component to be collected, etc.). When the system has been programmed, the operator phlebotomizes a donor (or otherwise accesses the blood of the blood source) and the system carries out the procedure, under the supervision of the operator.

The centrifuge rotates the separation chamber of the disposable flow circuit during processing, causing the heavier (greater specific gravity) components of the whole blood in the separation chamber, such as red blood cells, to move radially outwardly away from the center of rotation toward the outer or "high-G" wall of the separation chamber. The lighter (lower specific gravity) components, such as plasma, migrate toward the inner or "low-G" wall of the separation chamber. The boundary that forms between the heavier and lighter components in the separation chamber is commonly referred to as the interface. Depending on the speed at which the centrifuge is operated, a buffy coat may be formed between the plasma layer and the red blood cell layer, at the interface. The term "buffy coat" typically refers to the layer of platelets and white blood cells (which may include mononuclear cells and possibly peripheral blood stem cells) formed between a red blood cell layer and a plasma layer of separated whole blood, though it should be understood that some red blood cells and plasma will also be present in the buffy coat layer.

Various ones of these components can be selectively removed from the whole blood by providing appropriately located channeling structures and outlet ports in the flow circuit. For example, in one blood separation procedure, after the blood has been separated into plasma, buffy coat, and red blood cells, the buffy coat is collected, with plasma and red blood cells being returned to the blood source or separately collected.

A system capable of executing such a red blood cell, plasma, and buffy coat separation procedure is described in PCT Patent Application Publication No. WO 2021/194824 A1, which is hereby incorporated herein by reference in its entirety. More particularly, PCT Patent Application Publication No. WO 2021/194824 A1 describes a procedure in which blood in a continuous-flow centrifuge chamber is separated into plasma, buffy coat, and red blood cells. Separated plasma exits the chamber via a plasma outlet port and is collected in a plasma collection container, while separated red blood cells exit the chamber via a red blood cell outlet port and are collected in a red blood cell collection container. The buffy coat remains within the chamber while the other components are being collected.

During a buffy coat harvest stage, air from the plasma collection container is pumped into the chamber via the plasma outlet port. The air moves from the low-g wall of the chamber toward the high-g wall as additional air is conveyed into the chamber, which displaces the buffy coat remaining in the chamber through the red blood cell outlet port. The buffy coat is directed from the red blood cell outlet port into a buffy coat collection container, where it is collected as a buffy coat product.

Once a plurality of buffy coats has been collected (typically from different blood sources), they may be pooled together and subsequently separated by a centrifuge to produce a platelet product as described in U.S. Patent Application Publication No. 2018/0078582, which is incorporated herein by reference in its entirety. In U.S. Patent Application Publication No. 2018/0078582, the pooled buffy coats are received in a separation chamber and the centrifuge is operated at a rotation rate sufficient to separate the platelets from other cellular components of the buffy coats (e.g., red blood cells and white blood cells) in the separation chamber. The separated platelets are removed from the separation chamber and collected in a bag as the platelet product.

Although conventional approaches may effectively and reliably produce a platelet product, they may not be capable of removing a portion of the separated platelets from the separation chamber, which reduces the total volume of collected platelets in the bag.

### Summary

There are several aspects of the present subject matter which may be embodied separately or together in the devices and systems described and claimed below. These aspects may be employed alone or in combination with other aspects of the subject matter described herein, and the description of these aspects together is not intended to preclude the use of these aspects separately or the claiming of such aspects separately or in different combinations as set forth in the claims appended hereto.

In one aspect, a biological fluid processing device includes a pump system, a valve system, a centrifuge, and a controller. The controller is configured to execute a first platelet collection stage in which the pump system, the valve system, and the centrifuge are operated to convey buffy coat into the centrifuge, separate the buffy coat in the centrifuge into platelets and red blood cells, convey a first portion of the separated platelets from the centrifuge for collection, and convey the separated red blood cells from the centrifuge for collection. The controller is further configured to execute a second platelet collection stage in which the pump system, the valve system, and the centrifuge are operated to convey at least a portion of the collected red blood cells back into the centrifuge so as to convey a second portion of the platelets from the centrifuge for collection with the first portion of the separated platelets. The centrifuge is operated at a first rotation rate during the first platelet collection stage and at a lower second rotation rate during the second platelet collection stage.

In another aspect, a biological fluid processing system includes a reusable processing device and a disposable fluid flow circuit. The processing device includes a pump system, a valve system, a centrifuge and a controller, while the fluid flow circuit includes a processing chamber received by the centrifuge, a buffy coat container, a platelet container, a red blood cell container, and conduits fluidically connecting the processing chamber, the buffy coat container, the platelet container and the red blood cell container. The controller is configured to execute a first platelet collection stage in which the pump system, the valve system, and the centrifuge are operated to convey buffy coat from the buffy coat container into the processing chamber, separate the buffy coat in the processing chamber into platelets and red blood cells, convey a first portion of the separated platelets from the processing chamber via a first outlet line for collection in the platelet container and convey the separated red blood cells from the processing chamber via a second outlet line for collection in the red blood cell container. The controller is further configured to execute a second platelet collection stage in which the pump system, the valve system, and the centrifuge are operated to convey at least a portion of the collected red blood cells from the red blood cell container into the processing chamber via the second outlet line so as to convey a second portion of the separated platelets from the processing chamber via the first outlet line to the platelet container for collection with the first portion of the separated platelets. The centrifuge is operated at a first rotation rate during the first platelet collection stage and at a lower second rotation rate during the second platelet collection stage.

In yet another aspect, a method is provided for collecting platelets. The method includes conveying buffy coat into a centrifuge and then operating the centrifuge at a first rotation rate to separate the buffy coat into platelets and red blood cells. A first portion of the separated platelets is conveyed from the centrifuge for collection, with the separated red blood cells being conveyed from the centrifuge for collection. The centrifuge is then operated at a lower second rotation rate, with at least a portion of the collected red blood cells being conveyed back into the centrifuge so as to convey a second portion of the separated platelets from the centrifuge for collection.

In another aspect, a method is provided for collecting platelets. The method includes performing a first platelet collection stage in which a centrifuge is operated at a first rotation rate to separate buffy coat in the centrifuge into platelets and red blood cells, with a first portion of the separated platelets being collected from the centrifuge via a first outlet line. A second platelet collection stage is then performed, which includes operating the centrifuge at a lower second rotation rate, with a second portion of the separated platelets being collected from the centrifuge via the first outlet line.

### Brief Description of the Drawings

Fig. 1 is a perspective view of an exemplary reusable hardware component of a biological fluid processing system which is configured to receive a disposable fluid flow circuit;
Fig. 2 is a plan view of an exemplary disposable fluid flow circuit for use in combination with the durable hardware component of Fig. 1;
Fig. 3 is a schematic diagram of the reusable hardware component of Fig. 1 illustrating examples of various components connected to a controller;
Fig. 4 is a schematic view of the fluid flow circuit of Fig. 2 mounted to the reusable hardware component of Fig. 1 to complete a biological fluid processing system according to an aspect of the present disclosure;
Fig. 5 is a schematic view of the biological fluid processing system of Fig. 4 executing a "buffy coat prime" stage of an exemplary buffy coat separation procedure;
Fig. 6 is a schematic view of the biological fluid processing system of Fig. 4 executing an "establish separation" stage of an exemplary buffy coat separation procedure;
Fig. 7 is a schematic view of the biological fluid processing system of Fig. 4 executing a "steady-state separation" stage of an exemplary buffy coat separation procedure;
Fig. 8 is a schematic view of the biological fluid processing system of Fig. 4 executing a "platelet concentrate (PC) recovery" stage of an exemplary buffy coat separation procedure;
Fig. 9 is a block diagram of a method for platelet collection according to an embodiment of the present disclosure;
Fig. 10A is a front elevational view of an exemplary separation chamber of a fluid flow circuit; and
Fig. 10B is a bottom perspective view of the fluid flow path through the separation chamber of Fig. 10A.

### Description of the Illustrated Embodiments

The embodiments disclosed herein are for the purpose of providing a description of the present subject matter, and it is understood that the subject matter may be embodied in various other forms and combinations not shown in detail. Therefore, specific designs and features disclosed herein are not to be interpreted as limiting the subject matter as defined in the accompanying claims.

### Exemplary Biological Fluid Processing System

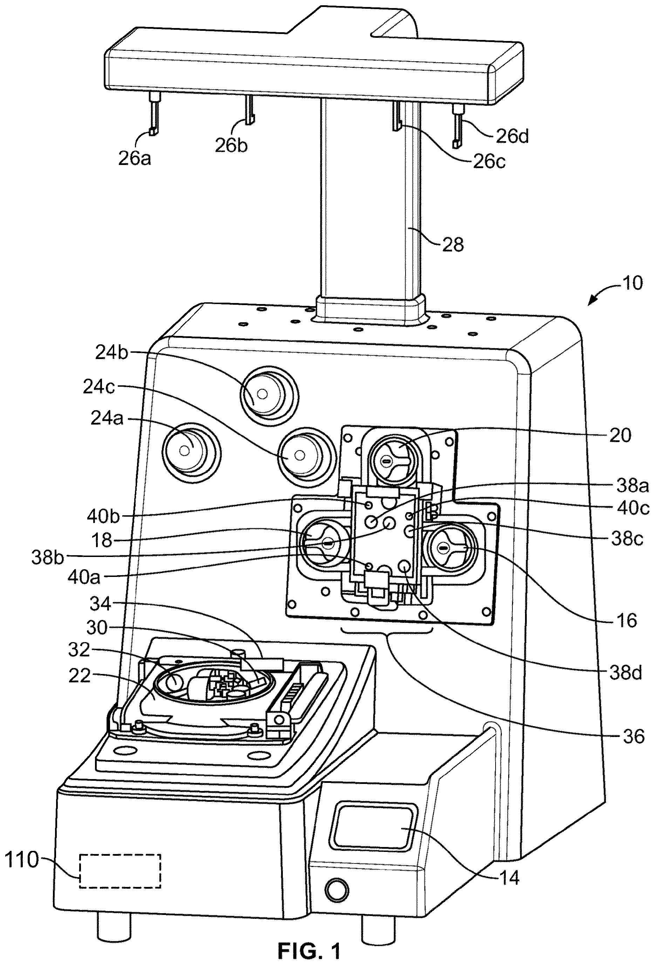

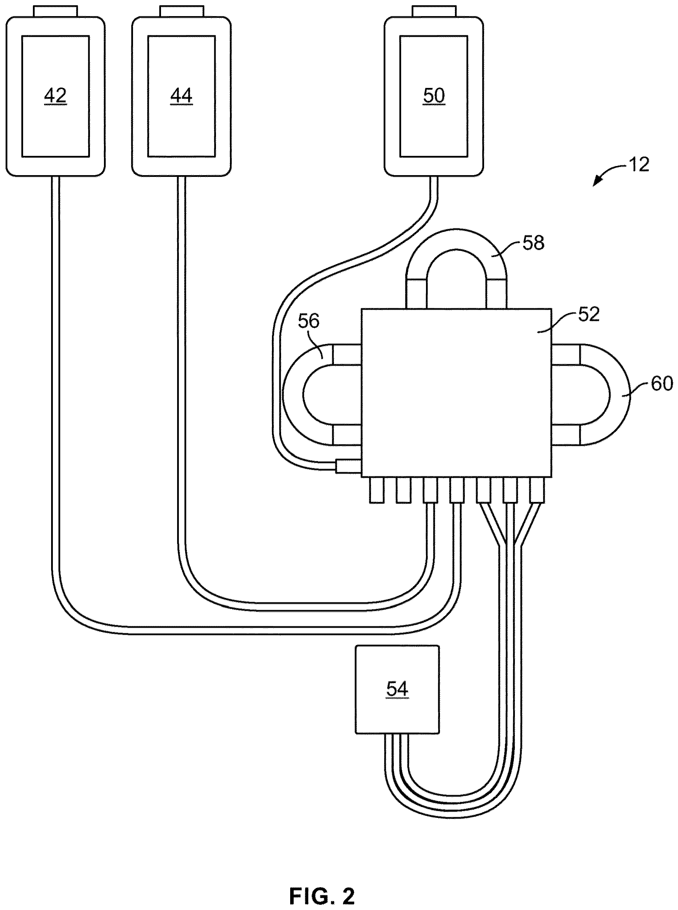

Fig. 1 depicts a reusable hardware component or processing device of a biological fluid processing system, generally designated 10, while Fig. 2 depicts a disposable fluid flow circuit, generally designated 12, to be used in combination with the processing device 10 for processing a biological fluid. While the illustrated processing device 10 may be used to process a variety of biological fluids (including whole blood), the present disclosure focuses on its ability to separate platelets from a pooled buffy coat for collection of the platelets as a platelet product. It is emphasized that the illustrated processing device 10 and fluid flow circuit 12 are merely exemplary and that differently configured centrifugal processing devices and fluid flow circuits may be employed without departing from the scope of the present disclosure.

The illustrated processing device 10 includes associated pumps, valves, sensors, displays, and other apparatus for configuring and controlling flow of fluid through the fluid flow circuit 12, described in greater detail below. The biological fluid processing system may be directed by a controller 110 integral with the processing device 10 that includes a programmable microprocessor to automatically control the operation of the pumps, valves, sensors, etc. The processing device 10 may also include wired or wireless communication capabilities to enable the transfer of data from the processing device 10 to a separate system (e.g., a quality management system of the operator) and/or to enable the transfer of data to the processing device 10 from such separate systems.

More specifically, the illustrated processing device 10 includes a user input and output touchscreen 14, a pump station including a first pump 16, a second pump 18 and a third pump 20, a centrifuge mounting station and drive unit 22 (which may be referred to herein as a "centrifuge"), and clamps 24a-c. The touchscreen 14 enables user interaction with the processing device 10, as well as the monitoring of procedure parameters, such as flow rates, container weights, pressures, etc. The pumps 16, 18, and 20 (collectively referred to herein as being part of a "pump system" of the processing device 10) are illustrated as peristaltic pumps capable of receiving tubing or conduits and moving fluid at various rates through the associated conduit dependent upon the procedure being performed. An exemplary centrifuge mounting station/drive unit is seen in U.S. Patent No. 8,075,468 (with reference to Figs. 26-28), which is hereby incorporated herein by reference. The clamps 24a-c (collectively referred to herein as being part of the "valve system" of the processing device 10) are capable of opening and closing fluid paths through the tubing or conduits and may incorporate RF sealers in order to complete a heat seal of the tubing or conduit placed in the clamp to seal the tubing or conduit leading to a product container upon completion of a procedure.

Sterile connection/docking devices may also be incorporated into one or more of the clamps 24a-c. The sterile connection devices may employ any of several different operating principles. For example, known sterile connection devices and systems include radiant energy systems that melt facing membranes of fluid flow conduits, as in U.S. Patent No. 4,157,723; heated wafer systems that employ wafers for cutting and heat bonding or splicing tubing segments together while the ends remain molten or semi-molten, such as in U.S. Patent Nos. 4,753,697; 5,158,630; and 5,156,701; and systems employing removable closure films or webs sealed to the ends of tubing segments as described, for example, in U.S. Patent No. 10,307,582. Alternatively, sterile connections may be formed by compressing or pinching a sealed tubing segment, heating and severing the sealed end, and joining the tubing to a similarly treated tubing segment as in, for example, U.S. Patent Nos. 10,040,247 and 9,440,396. All of the above-identified patents are incorporated by reference in their entirety. Sterile connection devices based on other operating principles may also be employed without departing from the scope of the present disclosure.

The processing device 10 also includes hangers 26a-d (which may each be associated with a weight scale) for suspending the various containers of the disposable fluid circuit 12. The hangers 26a-d are preferably mounted to a support 28, which is vertically translatable to improve the transportability of the processing device 10. An optical system comprising a laser 30 and a photodetector 32 is associated with the centrifuge 22 for determining and controlling the location of an interface between separated fluid components within the centrifuge 22. An exemplary optical system is shown in U.S. Patent Application Publication No. 2019/0201916, which is hereby incorporated herein by reference. An optical sensor 34 is also provided to optically monitor one or more conduits leading into or out of the centrifuge 22.

The face of the processing device 10 includes a nesting module 36 for seating a flow control cassette 52 (Fig. 2) of the fluid flow circuit 12. The cassette nesting module 36 is configured to receive various disposable cassette designs so that the system may be used to perform different types of procedures. Embedded within the illustrated cassette nesting module 36 are valves 38a-d (collectively referred to herein as being part of the "valve system" of the processing device 10, together with clamps 24a-c) for opening and closing fluid flow paths within the flow control cassette 52, and pressure sensors 40a-c capable of measuring the pressure at various locations of the fluid flow circuit 12.

With reference to Fig. 2, the illustrated fluid flow circuit 12 includes a plurality of containers 42, 44, 50, a flow control cassette 52 and a separation/processing chamber 54 configured to be received in the centrifuge 22, all of which are interconnected by conduits or tubing segments, so as to permit continuous flow centrifugation. The flow control cassette 52 routes the fluid flow through tubing loops 56, 58, 60, with each loop being positioned to engage a particular one of the pumps 16, 18, 20. The conduits or tubing may extend through the cassette 52, or the cassette 52 may have pre-formed fluid flow paths that direct the fluid flow.

In accordance with aspects of the present description, the fluid flow circuit is provided as a disposable buffy coat processing kit. As shown in Fig. 2, containers 42 and 44, may initially be empty containers for receiving platelet concentrate (i.e., separated platelets) and separated red blood cells, respectively, both of which have been separated from a pooled buffy coat using the centrifuge 22. With further reference to Fig. 2, the container 50 may be filled with buffy coat and connected to the fluid flow circuit 12 at the time of use. The buffy coat in container 50 may be pooled buffy coats extracted from multiple whole blood samples.

The processing chamber 54 may be pre-formed in a desired shape and configuration by injection molding from a rigid plastic material, as shown and described in U.S. Patent No. 6,849,039, which is hereby incorporated herein by reference. The specific geometry of the processing chamber 54 may vary depending on the elements to be separated, and the present disclosure is not limited to the use of any specific chamber design. For example, it is within the scope of the present disclosure for the processing chamber 54 to be configured formed of a generally flexible material, rather than a generally rigid material. When the processing chamber 54 is formed of a generally flexible material, it relies upon the centrifuge 22 to define a shape of the processing chamber 54. An exemplary processing chamber formed of a flexible material and an associated centrifuge are described in U.S. Patent No. 6,899,666, which is hereby incorporated herein by reference. Alternatively, or in addition, the processing chamber 54, according to some examples, may be formed similarly or identical to a centrifugal separation chamber shown and described in U.S. Patent No. 11,465,160, which is incorporated herein by reference in its entirety.

The controller 110 of the processing device 10 may be pre-programmed to automatically operate the system to perform one or more standard biological fluid processing procedures selected by an operator by input to the touchscreen 14. The controller 110 may be pre-programmed to substantially automate a wide variety of procedures, including, but not limited to: red blood cell and plasma production from a single unit of whole blood, buffy coat pooling, buffy coat separation into a platelet product (as described in U.S. Patent Application Publication No. 2018/0078582, which is hereby incorporated herein by reference), glycerol addition to red blood cells, red blood cell washing, platelet washing, and cryoprecipitate pooling and separation. The controller 110 may also be programmed by an operator in real-time to perform additional biological fluid processing procedures.

The pre-programmed biological fluid processing procedures or operations are associated with various settings, including operating parameters, for the components (e.g., pumps, clamps, valves, centrifuge etc.) of the processing device 10 which may be stored by the controller 110. Accordingly, selecting a pre-programmed biological fluid processing procedure causes the processing device 10, and in particular the various components of the processing device 10, to operate at the settings associated with the selected pre-programmed biological fluid processing procedure. Examples of such settings include but are not limited to positions, flow rates and centrifugation forces (or rotation rates). The controller 110 may be further configured to receive input from the operator as to one or more settings, such as flow rates and centrifugation forces, for the standard biological fluid processing procedure to override the pre-programmed settings. In addition, the controller 110 may be configured to receive input from the operator through the touchscreen 14 for operating the system to perform a non-standard biological fluid processing procedure.

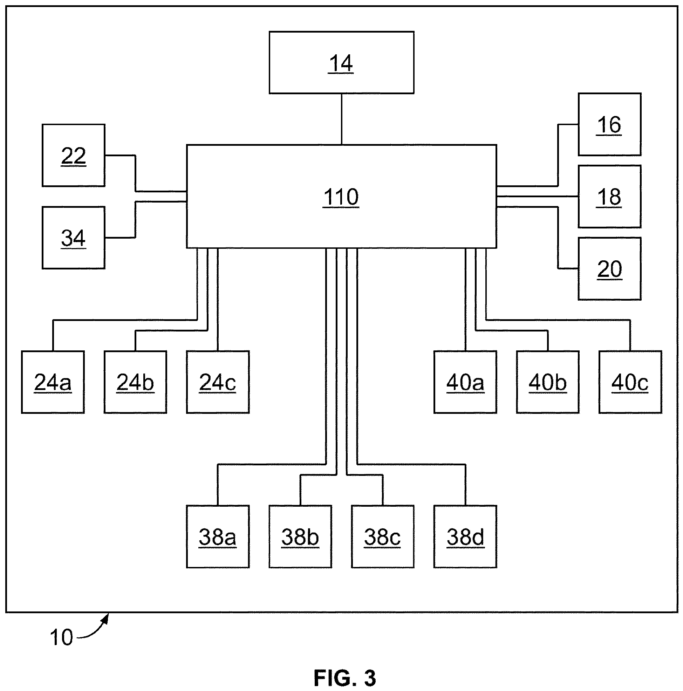

With reference to Fig. 3, the controller 110 is operably connected to various components of the processing device 10 including, for example, pumps 16, 18, 20, centrifuge 22, clamps 24a-c, and valves 38a-d, and operates the components (e.g., actuate, start, stop, and/or set, change, or maintain operating parameters such as positions, rotation rates, duration, intervals, flow rates etc. of the components) to perform a biological fluid processing operation. The controller 110 may also be connected to various sensors of the processing device 10 including, for example, optical sensor 34 and pressure sensors 40a-c, and may be configured to receive sensor information from the various sensors. The controller 110 is also connected to the touchscreen 14 and is configured to receive information input to the touchscreen 14 for example, by the operator, and output information to the touchscreen for example, to be displayed to the operator. It will be appreciated that the controller 110 may be connected to additional components and sensors of the processing device 10 not shown in the diagram of Fig. 3 or specifically referenced in the following examples, and further, may be configured to operate such components and/or receive sensor information from such sensors. In some examples, the controller 110 is configured to operate one or more components of the processing device 10 based on the sensor information received from the various sensors.

The controller 110 may control respective pumps 16, 18, 20 for example, to start or stop (i.e., to activate or deactivate) and to operate at various flow rates to convey a fluid through the fluid flow circuit 12. The controller 110 may control the centrifuge 22 to operate at various rotation rates to provide varying centrifugation forces and/or for various time intervals to process a fluid in the processing chamber 54. In addition, the controller 110 may control respective clamps 24a-c and respective valves 38a-d to move between open and closed positions to define a flow path in the fluid flow circuit 12.

The controller 110 is configured to receive optical information from the optical sensor 34 indicative of one or more optical characteristics of a fluid detected by the optical sensor 34. The controller 110 is also configured to receive pressure information from the pressure sensors 40a-c indicative of a fluid pressure detected by the pressure sensors 40a-c. Although not illustrated, the processing device 10 may include one or more weight scales or sensors operably connected to the controller 110 and configured to detect weights of one or more fluid containers 42, 44, 50. The controller 110 is configured to receive weight information from the weight sensors indicative of the weight detected by corresponding weight sensors.

The controller 110 may include a programmable microprocessor and a memory coupled to the microprocessor. The memory is a non-transitory computer readable medium and is configured to store program instructions to be executed by the microprocessor. The microprocessor is configured to perform one or more operations in response to executing the program instructions. For example, the microprocessor may control one or more of the various components (e.g., pumps 16, 18, 20, centrifuge 22, clamps 24a-c, valves 38a-d) to operate at a setting or parameter associated with a selected biological fluid processing operation based on the program instructions associated with a selected biological fluid processing operation. The memory may also store settings at which the components may operate for different biological fluid processing operations. The memory may store sensor information as well, such as the optical information, pressure information and weight information referenced above. The memory may include one or more memory devices for storing the program instructions, settings, sensor information and other information which may be used by the processing device 10.

### Buffy Coat Separation And Platelet Collection

According to an aspect of the present disclosure, the processing device 10 and the fluid flow circuit 12 may be used in combination to process a volume of (previously collected) pooled buffy coats to produce a platelet product (i.e., a collected volume of platelet concentrate separated from the pooled buffy coats).

In the procedure to be described, platelets are collected from pooled buffy coats by operating the centrifuge 22 at different rotation rates. For example, once the fluid flow circuit 12 has been primed, buffy coat may be conveyed to the processing chamber 54 while the centrifuge 22 is being operated at a first rotation rate. The first rotation rate provides centrifugation forces sufficient for separating the buffy coat into different components, namely a platelet fraction, a white blood cell fraction, and a red blood cell fraction. The separated platelets may be conveyed from the processing chamber 54 and collected in the platelet container 42, while the separated red blood cells may be conveyed from the processing chamber 54 and collected in the red blood cell container 44 (as a waste product). A majority of the white blood cells from the buffy coat will be sequestered in the processing chamber 54 during this stage, with such cells being later discarded as a waste product.

Subsequently, for example, after the buffy coat container 50 is emptied, the centrifuge 22 may be operated at one or more second rotation rates, lower than the first rotation rate, with any separated platelets remaining in the processing chamber 54 at the end of the previous stage being conveyed from the processing chamber 54 and collected in the platelet container 42 (with the platelets collected during the previous stage). Compared to conventional techniques, this approach allows for more complete collection of platelets from the separated buffy coat.

### Exemplary Buffy Coat Separation Procedure

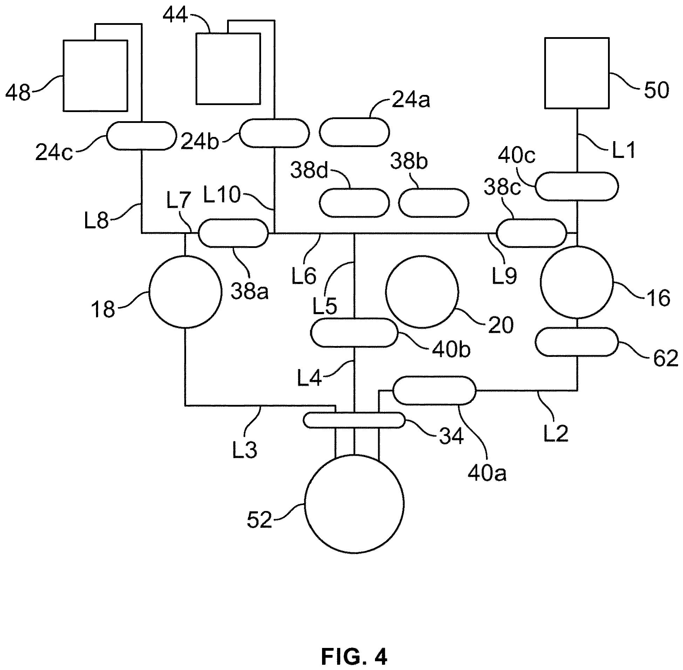

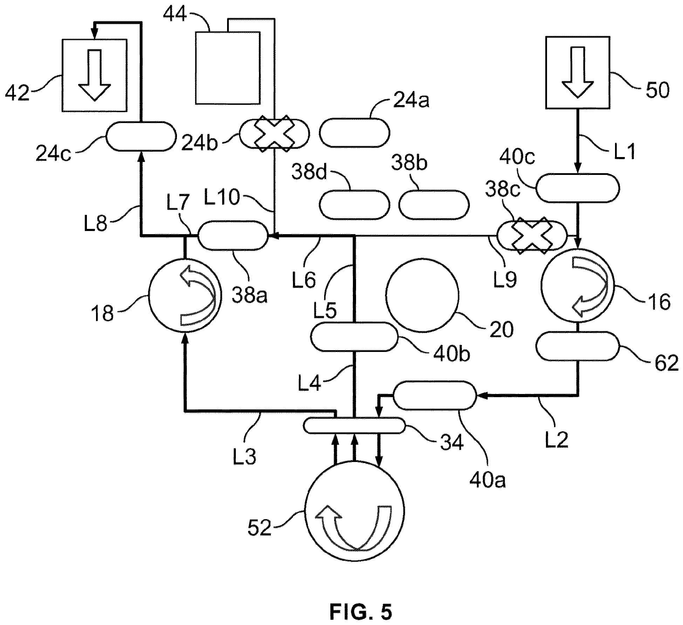

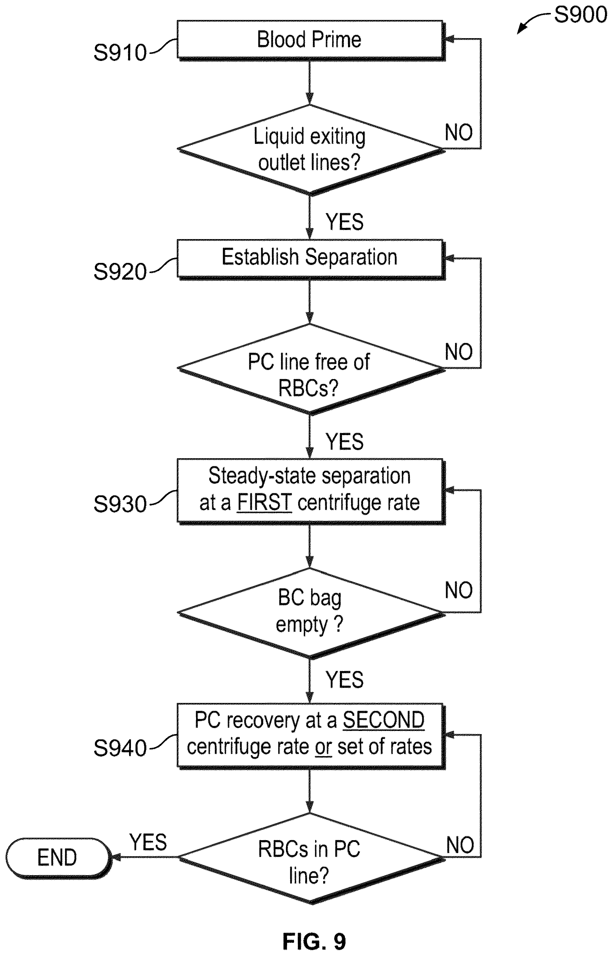

Fig. 4 is a schematic illustration of the fluid flow circuit 12 mounted to the processing device 10, with selected components of the fluid flow circuit 12 and selected components of the processing device 10 being shown. Figs. 5-8 show different stages of an exemplary procedure for buffy coat separation and platelet collection, while Fig. 9 illustrates an overview of the procedure.

In general, the biological fluid processing device 10 can be operated in different stages to collect platelets according to the present examples, with components of the biological fluid processing device being operated according to settings for such stages. However, as shown in Fig. 4, some components of the processing device 10 may remain "open" or unused, in that such components are not interfaced with a corresponding portion of the fluid flow circuit 12 used in the selected biological fluid processing operation. In the illustrated example, with the disposable buffy coat processing kit 12 arranged on the processing device 10, the third pump 20, first clamp 24a, second valve 38b and fourth valve 38d are not interfaced with the disposable buffy coat processing kit 12, and thus, do not affect fluid flow through the kit 12.

### "Buffy Coat Prime" Stage

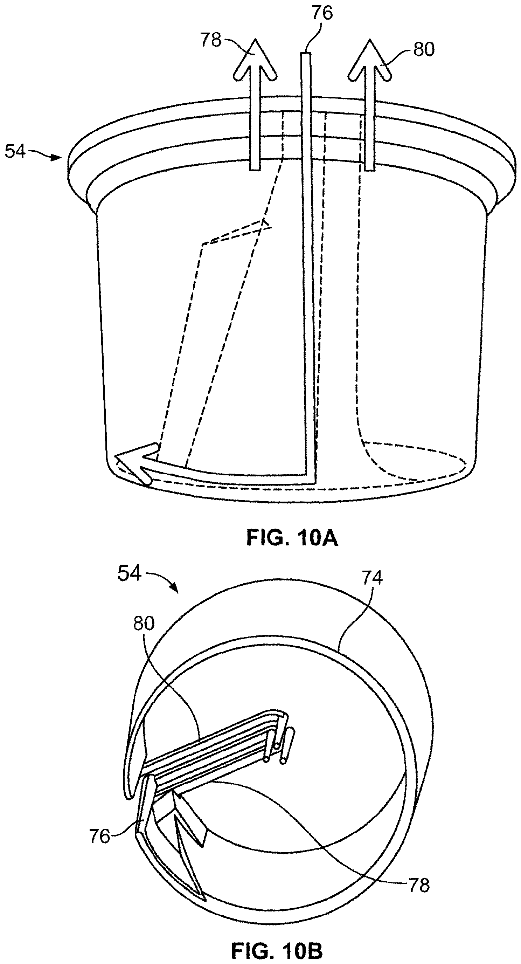

Referring now to Figs. 5, 10A and 10B, in a "buffy coat prime" stage, selected components of the fluid flow circuit 12 are primed to remove air therefrom. While it is within the scope of the present disclosure for a separately provided fluid (e.g., anticoagulant or saline) to be used for priming the fluid flow circuit 12, the prime stage of the illustrated example uses the buffy coat from the buffy coat container 50 for priming regions of the fluid flow circuit 12. Thus, in contrast to priming stages for typical apheresis devices, the system of the present examples does not require a separate solution (e.g., anticoagulant or saline) for priming regions of the fluid flow circuit 12.

During this stage, the controller 110 operates the pump and valve systems to convey buffy coat from the buffy coat container 50 into the processing chamber 54 positioned within the centrifuge 22 via an inlet or inlet passageway 76. This is done by closing valve 38c and actuating pump 16, which directs the buffy coat into the processing chamber 54 via lines L1, L2, third pressure sensor 40c, air trap 62, first pressure sensor 40a, optical sensor 34 and inlet 76. This pushes air from lines L1 and L2 into the processing chamber 54.

During the buffy coat prime stage, the centrifuge 22 may be operated at a rotation rate that is sufficiently low that it does not cause the buffy coat in the processing chamber 54 to be separated into its constituents. While it is also within the scope of the present disclosure for the centrifuge 22 to be inactive during this stage, operating the centrifuge 22 at a relatively low rate (e.g., in the range of approximately 1,000-2,000 rpm) may be preferable to the centrifuge 22 remaining inactive so as to decrease the risk of an air blockage in an umbilicus connected to the processing chamber 54.

A first portion of (unseparated) buffy coat pushes air from the centrifuge 22 via outlet line L3 (which is connected to a low-G outlet port 78 of the processing chamber 54) and a second portion of (unseparated) buffy coat pushes air from the processing chamber 54 via outlet line L4 (which is connected to a high-G outlet port 80 of the processing chamber 54). This includes air initially present in the processing chamber 54, along with air recently conveyed into the processing chamber 54 from lines L1 and L2. The percentage of the buffy coat flowing toward each outlet line depends upon the rate at which pump 18 is operated by the controller 110. More particularly, the second pump 18 (which is associated with line L3) is operated at a rate that is less than the rate at which the first pump 16 is operated, while there is no pump associated with line L4. By such a configuration, buffy coat will flow toward line L3 at a rate equal to the rate at which the second pump 18 is operated, while the remainder of the buffy coat will flow toward line L4 at a rate equal to the difference between the operational rates of the first and second pumps 16 and 18 (in accordance with basic fluid dynamics and conservation of mass principles). In a non-limiting example, the first pump 16 is operated at a first flow rate of about 100 ml/min and the second pump 18 is operated at a second flow rate of about 50 ml/min. Accordingly, buffy coat is conveyed into the processing chamber 54 according to the first flow rate (approximately 100 ml/min), the first portion of buffy coat is conveyed toward line L3 according to the second flow rate (approximately 50 ml/min), and the second portion of buffy coat is conveyed toward line L4 at a flow rate corresponding to the difference between the first flow rate and the second flow rate (approximately 50 ml/min).

Valve 38a is operated by the controller 110 to be open during this stage, while clamp 24b is operated by the controller 110 to be closed. As described above, valve 38c is also closed during this stage, which causes the air in line L4 to flow into lines L5, L6, and L7, through open valve 38a, and to a junction of lines L3 and L7, where it joins with the air flowing through line L3. The combined volumes of air flow into line L8, through open clamp 24c, and into the platelet container 42.

It should be understood that, in Figs. 5-8, arrows on the containers 42, 44, 50 represent the direction of fluid flow between the container and the conduit connected to the container. For example, with reference to Fig. 5, line L8 is shown as being connected to the top of the platelet container 42, such that a downward arrow (as in Fig. 5) represents downward fluid flow into the platelet container 42. In contrast, line L1 is shown as being connected to the bottom of the buffy coat container 50, such that a downward arrow (as in Fig. 5) represents downward fluid flow out of the buffy coat container 50.

The buffy coat prime stage may be performed until the presence of air is not detected in first outlet line L3 and second outlet line L4 (i.e., when buffy coat is detected exiting the processing chamber 54). The controller 110 may determine that air is not present in lines L3, L4 based on optical information received from optical sensor 34, and in response to determining air is not present, the controller 110 may be configured to end the buffy coat prime stage and transition to the next stage of the procedure. This is represented in Fig. 9 at S910, with the controller 110 periodically or continuously assessing whether air or buffy coat is exiting the processing chamber 54 and moving to a subsequent stage S920 when buffy coat is detected by the optical sensor 34.

### "Establish Separation" Stage

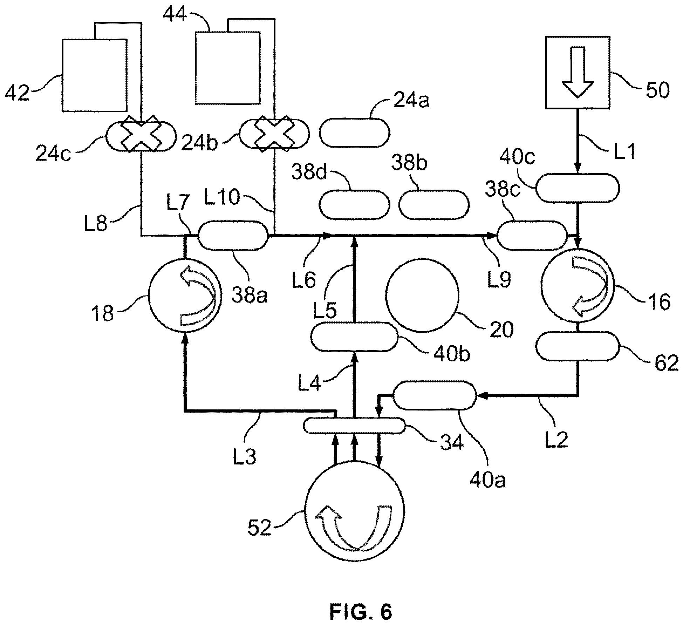

Referring now to Figs. 6, 10A and 10B, in the establish separation stage, the centrifuge 22 is operated at a rotation rate that is sufficient to separate the buffy coat into platelets (i.e., platelet concentrate) and red blood cells. The establish separation stage is intended to develop a platelet fraction that is substantially free of red blood cells and a red blood cell fraction that is substantially free of platelets, which includes moving the interface between the two fractions to a target location within the processing chamber 54. The exact location of the target interface position may vary without departing from the scope of the present disclosure (being at a location in which the platelet fraction occupies one half of the width of the channel 74 of the processing chamber 54 and the red blood cell fraction occupies the other half of the width of the channel, in one exemplary embodiment), provided that substantially pure platelet and red blood cell fractions are produced. Stated differently, the establish separation stage is executed so as to ensure that separated platelets (and not red blood cells) are flowing out of the processing chamber 54 via the low-G outlet port 78 and first outlet line L3 and that separated red blood cells (and not platelets) are flowing out of the processing chamber 54 via the high-G outlet port 80 and second outlet line L4 in preparation for subsequent platelet collection.

To perform the establish separation stage, the first pump 16 continues to operate (as in the buffy coat prime stage) to convey buffy coat out of the buffy coat container 50 and into the processing chamber 54 via lines L1 and L2, third pressure sensor 40c, air trap 62, first pressure sensor 40a, optical sensor 34 and inlet 76. The first pump 16 may operate at the same rate as it did during the buffy coat prime stage, at a greater rate, or at a lower rate (e.g., with the operational rate being decreased from 100 ml/min during the buffy coat prime stage to 60 ml/min during the establish separation stage).

As indicated above, during the establish separation stage, the centrifuge 22 is operated at a rotation rate that is sufficient for separating the buffy coat in the processing chamber 54 into a platelet fraction and a red blood cell fraction (i.e., at a greater rotation rate than in the buffy coat prime stage). In a non-limiting example, the centrifuge 22 may be operated at approximately 4,500 rpm (compared to operating at approximately 1,000-2,000 rpm during the buffy coat prime stage).

At this greater rotation rate (and greater g-force), the platelets of the buffy coat will begin to separate from the red blood cells within processing chamber 54. The platelets begin moving toward the low-G wall of the channel 74 of the processing chamber 54 (and the associated low-G outlet port 78 and first outlet line L3), while the red blood cells begin moving toward the high-G wall of the channel 74 (and the associated high-G outlet port 80 and second outlet line L4).

As during the buffy coat prime stage, the second pump 18 is operated during the establish separation stage to draw fluid from the processing chamber 54 via the first outlet line L3. Whereas unseparated buffy coat is pumped from the processing chamber 54 via the first outlet line L3 during the buffy coat prime stage, separated platelets are instead pumped from the processing chamber 54 via the first outlet line L3 and low-G outlet port 78 during the establish separation stage. As in the buffy coat prime stage, the remainder of the fluid in the processing chamber 54 will exit the processing chamber 54 via the second outlet line L4 and high-G outlet port 80 at a rate that is equal to the difference between the operational rates of the first pump 16 and the second pump 18 (due to basic fluid dynamics and conservation of mass principles). Whereas unseparated buffy coat is conveyed out of the processing chamber 54 via the second outlet line L4 during the buffy coat prime stage, separated red blood cells will instead flow out of the processing chamber 54 via the second outlet line L4 and associated high-G outlet port 80 during the establish separation stage.

Upon transitioning from the buffy coat prime stage to the establish separation stage, the controller 110 will cause clamp 24c to close, while causing valve 38c to open. Clamp 24b remains closed during the establish separation stage. Due to this change in the orientation of the valve system, the fluid (separated platelets) flowing through line L3 will flow into line L7 (instead of flowing into line L8), through open valve 38a, and into line L6. As during the buffy coat prime stage, the fluid (separated red blood cells) flowing through line L4 will flow into and through line L5 and to the junction of lines L5 and L6.

At the junction of lines L5 and L6, the fluid flowing through line L6 (separated platelets) will mix with the fluid flowing through line L5 (separated red blood cells) to form reconstituted buffy coat. Due to valve 38c being open during this stage, the reconstituted buffy coat will flow into and through line L9, through valve 38c to a junction where line L9 meets line L1, and then back into the processing chamber 54. Thus, it will be seen that the buffy coat is effectively recirculated through the processing chamber 54 during the establish separation stage.

At the beginning of the establish separation stage, the purity of the platelet fraction and red blood cell fraction may not be at the desired levels, so the establish separation stage will continue until the target purity level is achieved. This may be done by adjusting the operational rate of the second pump 18, either by increasing its speed (to reduce the thickness of the platelet fraction in the channel 74 of the processing chamber 54 and move the interface toward the low-G wall) or decreasing its speed (to increase the thickness of the platelet fraction in the channel 74 of the processing chamber 54 and move the interface toward the high-G wall).

The controller 110 may employ the optical sensor 34 to determine when steady-state separation of the buffy coat in the processing chamber 54 has been achieved. According to conventional techniques, the optical sensor 34 may monitor optical characteristics of fluid in one or both of outlet lines L3 and L4 and transmit detected optical information to the controller 110. The controller 110 may analyze the optical information provided by the optical sensor 34 to determine whether the platelet concentrate in line L3 is free from red blood cells. The controller 110 may additionally or alternatively analyze optical information regarding fluid flowing through line L4 to ascertain the purity of the red blood cells. However, as red blood cells are typically treated as a waste product, while the platelets are collected for later use as a platelet product, it may be preferable to at least monitor the status of the platelet concentrate flowing through line L3 to ensure that it is sufficiently clear of red blood cells before determining that steady-state separation has been achieved.

Once the controller 110 has determined (according to any suitable metric and approach) that steady-state separation has been achieved, it may advance the procedure to the next stage. This is represented in Fig. 9 at S920, with the controller 110 periodically or continuously assessing whether red blood cells are present in outlet line L3 (the "PC" line) and moving to a subsequent stage S930 when red blood cells are no longer present (or are at least present in a sufficiently low amount).

### "Steady-State Separation" Or "First Platelet Collection" Stage

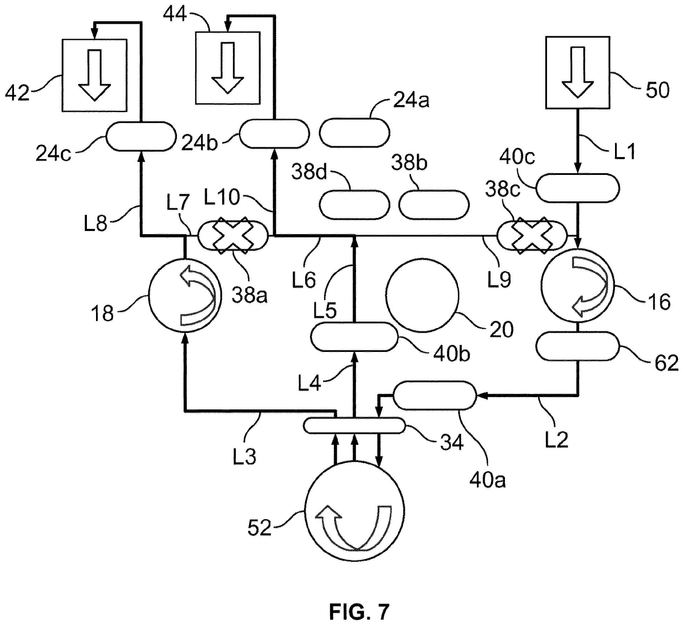

Fig. 7 illustrates an example of a system state suitable for performing a first platelet collection, or steady-state separation, stage. It will be seen that the centrifuge 22 and pumps 16 and 18 continue operating, while clamps 24b and 24c are opened, with valves 38a and 38c being closed.

During the first platelet collection stage, the pump and valve systems continue operating to convey buffy coat from the buffy coat container 50 into the centrifuge 22 (with the pumps 16 and 18 optionally operating at the same rates employed during the establish separation stage), with the centrifuge 22 being operated at a first rotation rate (which may be the same rate as employed during the establish separation stage, which is approximately 4,500 rpm in an exemplary embodiment) to separate the buffy coat into platelets and red blood cells.

Still referring to Fig. 7, and with additional reference to Figs. 10A and 10B, as during the establish separation stage, the buffy coat in the processing chamber 54 is separated into a platelet fraction and a red blood cell fraction, with the platelet fraction exiting the processing chamber 54 via the low-G outlet port 78 and first outlet line L3 and the red blood cell fraction exiting the processing chamber 54 via the high-G outlet port 80 and second outlet line L4. However, rather than being mixed as reconstituted buffy coat and recirculated back into the processing chamber 54 (as in the establish separation stage), a first portion of the separated platelets is instead directed into the platelet container 42 (via lines L3 and L8), while the separated red blood cells are directed from the centrifuge 22 into red blood cell container 44 (via lines L4, L5, L6, and L10).

As explained above, in addition to the platelets and red blood cells, the buffy coat also includes white blood cells. Preferably, a majority of the white blood cells present in the buffy coat container 50 are sequestered in the processing chamber 54 during this stage and may form waste to be discarded upon completion of the procedure.

The controller 110 may end the first platelet collection stage at any appropriate time. In one embodiment, the controller 110 is configured to end the first platelet collection stage upon determining that the buffy coat container 50 is empty or has otherwise reached a target weight or volume. The controller 110 may make this determination based on status information received from one or more sensors. The status information may be indicative of, for example, a detected weight of the buffy coat container 50 (when a weight scale measuring the weight of the buffy coat container 50 is coupled to the controller 110) and/or a detected hydrostatic pressure in a fluid line (line L1) exiting the buffy coat container 50 (when the controller 110 is receiving status information from the third pressure sensor 40c).

In response to determining that the buffy coat container 50 is empty or has reached the target weight or volume (or otherwise upon determining that it is appropriate to end the first platelet collection stage), the controller 110 may end the first platelet collection stage and advance the procedure to the next stage. This is represented in Fig. 9 at S930, with the controller periodically or continuously assessing whether the buffy coat container 50 is empty and moving to a subsequent stage S940 when the buffy coat container 50 is empty.

Notably, an amount of platelet concentrate will remain in the processing chamber 54 at the end of the first platelet collection stage as uncollected platelets.

### "Platelet Concentrate Recovery" Or "Second Platelet Collection" Stage

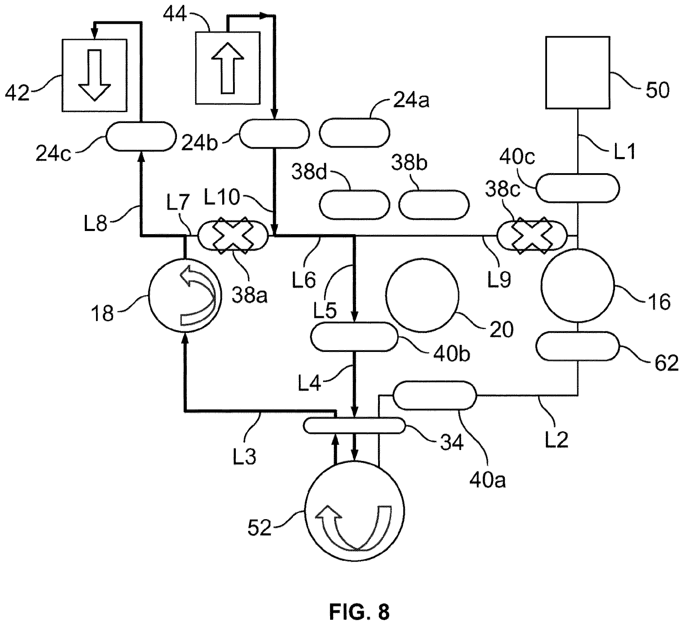

Fig. 8 illustrates the second platelet collection, or platelet concentrate recovery, stage, in which a second portion of the separated platelets (namely, the platelet concentrate remaining in the processing chamber 54 at the end of the first platelet collection stage) is collected.

Still referring to Fig. 8, and with additional reference to Figs. 10A and 10B, upon beginning the second platelet collection stage, the controller 110 will make no changes to the valve system, but will place the first pump 16 into an inactive state, such that only the second pump 18 is operational. Due to this change in the operation of the pump system, fluid (collected red blood cells) will be drawn out of the red blood cell container 44 via line L10 at a rate equal to the operational rate of the second pump 18. As shown in Fig. 8, the fluid (collected red blood cells) flows from line L10 into line L6 and then through lines L5 and L4, into the processing chamber 54 (via its high-G outlet port 80). The operational rate of the second pump 18 may vary without departing from the scope of the present disclosure, though it may be advantageous for it to be relatively low (e.g., 20 ml/min) to better control the flow of fluid through the fluid flow circuit 12 during this stage.

As the collected red blood cells continue to be drawn into the processing chamber 54 via the high-G outlet port 80, the thickness of the red blood cell layer within the channel 74 of the processing chamber 54 will increase. The inlet port 76 of the processing chamber 54 is functionally closed (due to the first pump 16 being inactive during this stage), such that any fluid exiting the processing chamber 54 must do so via the low-G outlet port 78 and associated first outlet line L3 (as shown in Fig. 8). As noted above, an amount of platelet concentrate will be present in the processing chamber 54 at the end of the first platelet collection stage and at the beginning of the second platelet collection stage. On account of these platelets being positioned radially inwardly of the red blood cells entering the processing chamber 54, they will be pushed out of the processing chamber by the returning red blood cells via the low-G outlet port 78 and associated first outlet line L3.

During the second platelet collection stage, the operational or rotation rate of the centrifuge 22 is reduced from the first rotation rate (i.e., the one employed during the first platelet collection stage, which is approximately 4,500 rpm in an exemplary embodiment) to a lower rotation rate (which is referred to herein as a "second" rotation rate). It should be understood that the exact rate at which the centrifuge 22 is operated during the second platelet collection stage may vary without departing from the scope of the present disclosure. Additionally, it should be understood that the centrifuge 22 need not be operated at the same rotation rate during the entirety of the second platelet collection stage, but rather one or more second rotation rates (each lower than the rotation rate employed during the first platelet collection stage) may be employed during the second platelet collection stage. For example, the centrifuge 22 may be operated at a constant second rotation rate of approximately 2,750 rpm during the entire second platelet collection stage. In other examples, the centrifuge 22 may be operated so as to gradually decrease in a linear manner from one rotation rate (which may be equal to or less than the rotation rate employed during the first platelet collection stage) to a lower rotation rate (e.g., by reducing the rotation rate from approximately 4,500 rpm to approximately 2,000 rpm). The rotation rate may also be varied in a non-linear manner (e.g., in a stepwise manner) during the second platelet collection stage.

While the exact rotation rate of the centrifuge 22 during the second platelet collection stage may vary without departing from the scope of the present disclosure, it may be advantageous for the centrifuge 22 to operate at a rotation rate that is sufficiently low such that it is no longer operating in a continuous flow separation mode (as it was operating during the establish separation and first platelet collection stages). When not operating in a continuous flow separation mode, the plasma required for elutriation is not produced, thereby causing the residence time of the platelets in the processing chamber 54 to increase. Reducing the rotation rate of the centrifuge 22 to a sufficiently low level ensures that that the platelets retained in the processing chamber 54 at the end of the first platelet collection stage will remain suspended in the plasma during the second platelet collection stage rather than settling towards the red blood cell bed (which gradually builds up within the channel 74 of the processing chamber 54 as the collected red blood cells are pulled back into the processing chamber 54 from the red blood cell container 44).

As shown in Fig. 8, the platelets (suspended in plasma) are forced from the processing chamber 54 via the low-G outlet port 78 and associated first outlet line L3 by the returning red blood cells, with the platelets flowing from line L3 into line L8 and then into the platelet container 42. In this manner, a platelet product is produced which includes platelet concentrate collected during a continuous flow separation mode while buffy coat is provided to the processing chamber 54 (i.e., during the first platelet collection stage), as well platelet concentrate remaining in the processing chamber 54 after the buffy coat container 50 is emptied (i.e., during the second platelet collection stage). On account of this second portion of platelets being collected, it will be seen that executing this second platelet collection stage allows for a more complete collection of platelets and a platelet product having a greater volume than is possible when using conventional techniques.

The controller 110 may end the second platelet collection stage at any suitable time, based on any appropriate criteria. In one embodiment, the controller 110 is configured to end the second platelet collection stage upon determining that red blood cells are present in the first outlet line L3, which is indicative of the platelets having been removed from the processing chamber 54 and collected in the platelet container 42 (due to the red blood cells following the platelets through the first outlet line L3 during this stage). The controller 110 may make this determination based on optical information received from the optical sensor 34, similar to the way in which optical information from the optical sensor 34 is used by the controller 110 during the establish separation stage to determine whether red blood cells are present in the first outlet line L3.

In response to determining that red blood cells are present in the first outlet line L3 (or otherwise upon determining that it is appropriate to end the second platelet collection stage), the controller 110 may end the second platelet collection stage and advance the procedure to the next stage or end the procedure (if there are no additional stages). This is represented in Fig. 9 at S940, with the controller periodically or continuously assessing whether red blood cells are present in the first outlet line L3 and ending the second platelet collection procedure when red blood cells have been detected there.

### ADDITIONAL STAGES

When the second platelet collection stage has been completed, the controller 110 may execute any of a number of subsequent stages. For example, an amount of air will remain in the platelet container 42 at the end of the procedure, on account of the air being pushed into the platelet container 42 during the buffy coat prime stage. In an optional stage, the second pump 18 may be operated in a reverse direction (compared to the direction in which it is operated during the separation and collection procedure) to pull air out of the platelet container 42.

In another optional stage, the controller 110 may actuate a sealing mechanism (e.g., one incorporated into or otherwise associated with the third clamp 24c) to seal line L8, which encloses the platelet product within the platelet container 42. Line L8 may be severed at the location of the seal to allow for the platelet container 42 to be separated from the remainder of the fluid flow circuit 12, which may be discarded as a waste product.

It should be understood that the processing device 10 shown in Figs. 1 and 3, the fluid flow circuit 12 shown in Figs. 2 and 4, and the procedures shown in Figs. 5-9 are merely exemplary of a system and procedure that may employ the techniques and principles described herein. Indeed, differently configured biological fluid separation systems and separation procedures including different steps may employ the techniques and principles described herein without departing from the scope of the present disclosure.

### Aspects

Aspect 1. A biological fluid processing device, comprising: a pump system; a valve system; a centrifuge; and a controller, wherein the controller is configured to execute a first platelet collection stage in which the pump system, the valve system, and the centrifuge are operated to convey buffy coat into the centrifuge, separate the buffy coat in the centrifuge into platelets and red blood cells, convey a first portion of the separated platelets from the centrifuge for collection, and convey the separated red blood cells from the centrifuge for collection, and execute a second platelet collection stage in which the pump system, the valve system, and the centrifuge are operated to convey at least a portion of the collected red blood cells back into the centrifuge so as to convey a second portion of the platelets from the centrifuge for collection with the first portion of the separated platelets, wherein the centrifuge is operated at a first rotation rate during the first platelet collection stage and at a lower second rotation rate during the second platelet collection stage.

Aspect 2. The biological fluid processing device of Aspect 1, wherein the pump system includes a first pump and a second pump, and the controller is configured to operate the first pump and the second pump during the first platelet collection stage to simultaneously convey the buffy coat into the centrifuge, the first portion of the separated platelets from the centrifuge and the separated red blood cells from the centrifuge.

Aspect 3. The biological fluid processing device of Aspect 2, wherein the controller deactivates the first pump and operates the second pump during the second platelet collection stage to simultaneously convey the collected red blood cells into the centrifuge and convey the second portion of the platelets from the centrifuge.

Aspect 4. The biological fluid processing device of any one of the preceding Aspects, wherein the controller is configured to operate the centrifuge at the first rotation rate when the pump system is operated to convey buffy coat into the centrifuge, convey the first portion of the separated platelets out of the centrifuge and convey the separated red blood cells out of the centrifuge.

Aspect 5. The biological fluid processing device of any one of the preceding Aspects, wherein the controller is configured to operate the centrifuge at a plurality of second rotation rates during the second platelet collection stage, with each second rotation rate being lower than the first rotation rate.

Aspect 6. A biological fluid processing system, comprising: a reusable processing device including a pump system, a valve system, a centrifuge and a controller; and a disposable fluid flow circuit including a processing chamber received by the centrifuge, a buffy coat container, a platelet container, a red blood cell container, and conduits fluidically connecting the processing chamber, the buffy coat container, the platelet container and the red blood cell container, wherein the controller is configured to execute a first platelet collection stage in which the pump system, the valve system, and the centrifuge are operated to convey buffy coat from the buffy coat container into the processing chamber, separate the buffy coat in the processing chamber into platelets and red blood cells, convey a first portion of the separated platelets from the processing chamber via a first outlet line for collection in the platelet container and convey the separated red blood cells from the processing chamber via a second outlet line for collection in the red blood cell container, and execute a second platelet collection stage in which the pump system, the valve system, and the centrifuge are operated to convey at least a portion of the collected red blood cells from the red blood cell container into the processing chamber via the second outlet line so as to convey a second portion of the separated platelets from the processing chamber via the first outlet line to the platelet container for collection with the first portion of the separated platelets, wherein the centrifuge is operated at a first rotation rate during the first platelet collection stage and at a lower second rotation rate during the second platelet collection stage.

Aspect 7. The biological fluid processing system of Aspect 6, wherein the reusable processing device further includes a buffy coat container sensor configured to detect a status of the buffy coat container and transmit status information indicative of the detected status to the controller, and during the first platelet collection stage, the controller is configured to determine whether the buffy coat container is empty based on the status information, and in response to determining that the buffy coat container is empty, end the first platelet collection stage and begin the second platelet collection stage

Aspect 8. The biological fluid processing system of Aspect 7, wherein the buffy coat container sensor is a weight sensor configured to detect a weight of the buffy coat container, and the status information is indicative of a detected weight of the buffy coat container.

Aspect 9. The biological fluid processing system of Aspect 7, wherein the buffy coat container sensor is a pressure sensor configured to detect a hydrostatic pressure in a line exiting the buffy coat container, and the status information is indicative of a detected hydrostatic pressure.

Aspect 10. The biological fluid processing system of any one of Aspects 6-9, wherein the reusable processing device further includes an optical sensor configured to detect optical characteristics of fluid exiting the processing chamber and transmit optical information indicative of the detected optical characteristics to the controller, and during the second platelet collection stage, the controller is configured to determine whether separated red blood cells are present in the first outlet line based on the optical information, and in response to determining that separated red blood cells are present in the first outlet line, end the second platelet collection stage.

Aspect 11. The biological fluid processing system of any one of Aspects 6-10, wherein the controller is further configured to execute a buffy coat prime stage prior to the first platelet collection stage, with the buffy coat prime stage including operating the pump system and the valve system to convey buffy coat from the buffy coat container into the processing chamber, operating the centrifuge at a third rotation rate lower than the first rotation rate, operating the pump system and the valve system to convey a first portion of unseparated buffy coat toward the first outlet line so as to convey air from the processing chamber to the platelet container via the first outlet line, and operating the pump system and the valve system to convey a second portion of unseparated buffy coat toward the second outlet line so as to convey air from the processing chamber to the platelet container via the second outlet line.

Aspect 12. The biological fluid processing system of Aspect 11, wherein the controller is further configured to execute an establish separation stage after the biological fluid prime stage and prior to the first platelet collection stage, with the establish separation stage including operating the pump system and the valve system to convey buffy coat from the buffy coat container to the processing chamber, operating the centrifuge to separate the buffy coat in the processing chamber into platelets and red blood cells, operating the pump system and the valve system to convey the separated platelets from the processing chamber via the first outlet line, operating the pump system and the valve system to convey the separated red blood cells from the processing chamber via the second outlet line, and operating the pump system and the valve system to combine the separated platelets and the separated red blood cells as reconstituted buffy coat and convey the reconstituted buffy coat back into the processing chamber.

Aspect 13. The biological fluid processing system of Aspect 12, wherein the reusable processing device further includes a buffy coat container sensor configured to detect a status of the buffy coat container and transmit status information indicative of the detected status to the controller, and an optical sensor configured to detect optical characteristics of fluid exiting the processing chamber and transmit optical information indicative of the detected optical characteristics to the controller.

Aspect 14. The biological fluid processing system of Aspect 13, wherein: during the biological fluid prime stage, the controller is configured to determine whether air is present in the first outlet line or the second outlet line based on the optical information, and in response to determining that air is not present in either the first outlet line or the second outlet line, end the biological fluid prime stage and begin the establish separation stage, during the establish separation stage, the controller is configured to determine whether separated red blood cells are present in the first outlet line based on the optical information, and in response to determining that separated red blood cells are not present in the first outlet line, end the establish separation stage and begin the first platelet collection stage, during the first platelet collection stage, the controller is configured to determine whether the buffy coat container is empty based on the status information, and in response to determining that the buffy coat container is empty, end the first platelet collection stage and begin the second platelet collection stage, and during the second platelet collection stage, the controller is configured to determine whether separated red blood cells are present in the first outlet line based on the optical information, and in response to determining that separated red blood cells are present in the first outlet line, end the second platelet collection stage.

Aspect 15. A method of collecting platelets comprising: conveying buffy coat into a centrifuge; operating the centrifuge at a first rotation rate to separate the buffy coat into platelets and red blood cells; conveying a first portion of the separated platelets from the centrifuge for collection; conveying the separated red blood cells from the centrifuge for collection; operating the centrifuge at a lower second rotation rate; and conveying at least a portion of the collected red blood cells back into the centrifuge so as to convey a second portion of the separated platelets from the centrifuge for collection.

Aspect 16. A method of collecting platelets comprising: performing a first platelet collection stage in which a centrifuge is operated at a first rotation rate to separate buffy coat in the centrifuge into platelets and red blood cells, wherein a first portion of the separated platelets are collected from the centrifuge via a first outlet line; and performing a second platelet collection stage in which the centrifuge is operated at a lower second rotation rate, wherein a second portion of the separated platelets are collected from the centrifuge via the first outlet line.

Aspect 17. The method of Aspect 16, further comprising determining whether a buffy coat container fluidically connected to the centrifuge is empty, and performing the second platelet collection stage in response to determining that the buffy coat container is empty.

Aspect 18. The method of any one of Aspects 16-17, further comprising determining whether separated red blood cells are present in the first outlet line during the second platelet collection stage; and ending the second platelet collection stage in response to determining that separated red blood cells are present in the first outlet line.

Aspect 19. The method of any one of Aspects 16-18, further comprising performing a biological fluid prime stage to remove air from the centrifuge via the first outlet line and a second outlet line prior to performing the first platelet collection stage, and performing an establish separation stage to remove red blood cells from the first outlet line after performing the biological fluid prime stage and prior to performing the first platelet collection stage.

Aspect 20. The method of Aspect 19, further comprising determining whether air is present in the first outlet line or the second outlet line during the biological fluid prime stage and, upon determining that air is not present in the first outlet line or the second outlet line, ending the biological fluid prime stage and beginning the establish separation stage, determining whether separated red blood cells are present in the first outlet line during the establish separation stage and, upon determining that separated red blood cells are not present in the first outlet line, ending the establish separation stage and beginning the first platelet collection stage, determining whether a buffy coat container fluidically connected to the centrifuge is empty during the first platelet collection stage and, upon determining that the buffy coat container is empty, ending the first platelet collection stage and beginning the second platelet collection stage, and determining whether separated red blood cells are present in the first outlet line during the second platelet collection stage and, upon determining that separated red blood cells are present in the first outlet line, ending the second platelet collection stage.

It will be understood that the embodiments and examples described above are illustrative of some of the applications of the principles of the present subject matter. Numerous modifications may be made by those skilled in the art without departing from the spirit and scope of the claimed subject matter, including those combinations of features that are individually disclosed or claimed herein. For these reasons, the scope hereof is not limited to the above description but is as set forth in the following claims, and it is understood that claims may be directed to the features hereof, including as combinations of features that are individually disclosed or claimed herein.

## Claims

1. A biological fluid processing device, comprising:
a pump system;
a valve system;
a centrifuge; and
a controller, wherein the controller is configured to
execute a first platelet collection stage in which the pump system, the valve system, and the centrifuge are operated to convey buffy coat into the centrifuge, separate the buffy coat in the centrifuge into platelets and red blood cells, convey a first portion of the separated platelets from the centrifuge for collection, and convey the separated red blood cells from the centrifuge for collection, and
execute a second platelet collection stage in which the pump system, the valve system, and the centrifuge are operated to convey at least a portion of the collected red blood cells back into the centrifuge so as to convey a second portion of the platelets from the centrifuge for collection with the first portion of the separated platelets, wherein the centrifuge is operated at a first rotation rate during the first platelet collection stage and at a lower second rotation rate during the second platelet collection stage.

2. The biological fluid processing device of claim 1, wherein
the pump system includes a first pump and a second pump, and
the controller is configured to operate the first pump and the second pump during the first platelet collection stage to simultaneously convey the buffy coat into the centrifuge, the first portion of the separated platelets from the centrifuge and the separated red blood cells from the centrifuge.

3. The biological fluid processing device of claim 2, wherein the controller deactivates the first pump and operates the second pump during the second platelet collection stage to simultaneously convey the collected red blood cells into the centrifuge and convey the second portion of the platelets from the centrifuge.

4. The biological fluid processing device of any one of the preceding claims, wherein the controller is configured to operate the centrifuge at the first rotation rate when the pump system is operated to convey buffy coat into the centrifuge, convey the first portion of the separated platelets out of the centrifuge and convey the separated red blood cells out of the centrifuge.

5. The biological fluid processing device of any one of the preceding claims, wherein the controller is configured to operate the centrifuge at a plurality of second rotation rates during the second platelet collection stage, with each second rotation rate being lower than the first rotation rate.

6. A biological fluid processing system, comprising:
a reusable processing device including a pump system, a valve system, a centrifuge and a controller; and
a disposable fluid flow circuit including a processing chamber received by the centrifuge, a buffy coat container, a platelet container, a red blood cell container, and conduits fluidically connecting the processing chamber, the buffy coat container, the platelet container and the red blood cell container, wherein the controller is configured to
execute a first platelet collection stage in which the pump system, the valve system, and the centrifuge are operated to convey buffy coat from the buffy coat container into the processing chamber, separate the buffy coat in the processing chamber into platelets and red blood cells, convey a first portion of the separated platelets from the processing chamber via a first outlet line for collection in the platelet container and convey the separated red blood cells from the processing chamber via a second outlet line for collection in the red blood cell container, and
execute a second platelet collection stage in which the pump system, the valve system, and the centrifuge are operated to convey at least a portion of the collected red blood cells from the red blood cell container into the processing chamber via the second outlet line so as to convey a second portion of the separated platelets from the processing chamber via the first outlet line to the platelet container for collection with the first portion of the separated platelets, wherein the centrifuge is operated at a first rotation rate during the first platelet collection stage and at a lower second rotation rate during the second platelet collection stage.

7. The biological fluid processing system of claim 6, wherein
the reusable processing device further includes a buffy coat container sensor configured to detect a status of the buffy coat container and transmit status information indicative of the detected status to the controller, and
during the first platelet collection stage, the controller is configured to determine whether the buffy coat container is empty based on the status information, and in response to determining that the buffy coat container is empty, end the first platelet collection stage and begin the second platelet collection stage.

8. The biological fluid processing system of claim 6 or 7, wherein
the reusable processing device further includes an optical sensor configured to detect optical characteristics of fluid exiting the processing chamber and transmit optical information indicative of the detected optical characteristics to the controller, and
during the second platelet collection stage, the controller is configured to determine whether separated red blood cells are present in the first outlet line based on the optical information, and in response to determining that separated red blood cells are present in the first outlet line, end the second platelet collection stage.

9. The biological fluid processing system of any one of claims 6-8, wherein the controller is further configured to execute a buffy coat prime stage prior to the first platelet collection stage, with the buffy coat prime stage including
operating the pump system and the valve system to convey buffy coat from the buffy coat container into the processing chamber,
operating the centrifuge at a third rotation rate lower than the first rotation rate,
operating the pump system and the valve system to convey a first portion of unseparated buffy coat toward the first outlet line so as to convey air from the processing chamber to the platelet container via the first outlet line, and
operating the pump system and the valve system to convey a second portion of unseparated buffy coat toward the second outlet line so as to convey air from the processing chamber to the platelet container via the second outlet line.

10. The biological fluid processing system of claim 9, wherein the controller is further configured to execute an establish separation stage after the biological fluid prime stage and prior to the first platelet collection stage, with the establish separation stage including
operating the pump system and the valve system to convey buffy coat from the buffy coat container to the processing chamber,
operating the centrifuge to separate the buffy coat in the processing chamber into platelets and red blood cells,
operating the pump system and the valve system to convey the separated platelets from the processing chamber via the first outlet line,
operating the pump system and the valve system to convey the separated red blood cells from the processing chamber via the second outlet line, and
operating the pump system and the valve system to combine the separated platelets and the separated red blood cells as reconstituted buffy coat and convey the reconstituted buffy coat back into the processing chamber.

11. The biological fluid processing system of claim 10, wherein:
during the biological fluid prime stage, the controller is configured to determine whether air is present in the first outlet line or the second outlet line based on the optical information, and in response to determining that air is not present in either the first outlet line or the second outlet line, end the biological fluid prime stage and begin the establish separation stage,
during the establish separation stage, the controller is configured to determine whether separated red blood cells are present in the first outlet line based on the optical information, and in response to determining that separated red blood cells are not present in the first outlet line, end the establish separation stage and begin the first platelet collection stage,
during the first platelet collection stage, the controller is configured to determine whether the buffy coat container is empty based on the status information, and in response to determining that the buffy coat container is empty, end the first platelet collection stage and begin the second platelet collection stage, and
during the second platelet collection stage, the controller is configured to determine whether separated red blood cells are present in the first outlet line based on the optical information, and in response to determining that separated red blood cells are present in the first outlet line, end the second platelet collection stage.

12. A method of collecting platelets comprising:
conveying buffy coat into a centrifuge;
operating the centrifuge at a first rotation rate to separate the buffy coat into platelets and red blood cells;
conveying a first portion of the separated platelets from the centrifuge for collection;
conveying the separated red blood cells from the centrifuge for collection;
operating the centrifuge at a lower second rotation rate; and
conveying at least a portion of the collected red blood cells back into the centrifuge so as to convey a second portion of the separated platelets from the centrifuge for collection.

13. A method of collecting platelets comprising:
performing a first platelet collection stage in which a centrifuge is operated at a first rotation rate to separate buffy coat in the centrifuge into platelets and red blood cells, wherein a first portion of the separated platelets are collected from the centrifuge via a first outlet line; and
performing a second platelet collection stage in which the centrifuge is operated at a lower second rotation rate, wherein a second portion of the separated platelets are collected from the centrifuge via the first outlet line.

14. The method of claim 13, further comprising
determining whether a buffy coat container fluidically connected to the centrifuge is empty, and
performing the second platelet collection stage in response to determining that the buffy coat container is empty.

15. The method of claim 13 or 14, further comprising
determining whether separated red blood cells are present in the first outlet line during the second platelet collection stage; and
ending the second platelet collection stage in response to determining that separated red blood cells are present in the first outlet line.
